Europäisches Patentamt

**European Patent Office**

Office européen des brevets

(19)

(11) Numéro de publication: **0 265 325
B1**

(12) **FASCICULE DE BREVET EUROPEEN**

(45) Date de publication du fascicule du brevet:
**22.11.90**

(51) Int. Cl.⁵: **A61F 2/38**

(21) Numéro de dépôt: **87402271.8**

(22) Date de dépôt: **13.10.87**

(54) Prothèse d'articulation du genou.

(30) Priorité: **14.10.86 FR 8614251**

(43) Date de publication de la demande:
**27.04.88 Bulletin 88/17**

(45) Mention de la délivrance du brevet:
**22.11.90 Bulletin 90/47**

(84) Etats contractants désignés:
**AT BE CH DE GB IT LI NL SE**

(56) Documents cités:
**DE-A- 2 114 287
FR-A- 1 532 997
GB-A- 1 328 497**

**METAL PROGRESS MATERIAL & PROCESSING,
Databook 1981, vol. 120, no. 1, juin 1981, pages 90,91, American Society for Metal; "Cobalt-base
Alloys", "Elgiloy" et "MP35N"**

(73) Titulaire: **S.N.R. ROULEMENTS, Boîte Postale 17 1, rue
des Usines, F-74010 Annecy Cédex(FR)**

(72) Inventeur: **Peilloud, Fernand, Hery/Alby,
F-74540 Alby-Cheran(FR)**

(74) Mandataire: **Ernst-Schonberg, Michel et al, R.N.U.R.
S. 0804 B.P. 103 8 & 10, avenue Emile-Zola,
F-92109 Boulogne Billancourt(FR)**

## Description

L'invention concerne une prothèse d'articulation du genou selon la première partie de la revendication 1. Une prothèse de ce type est décrit dans la publication GB-A 1 328 497.

La publication FR-A 2 330 375 décrit aussi une telle prothèse étudiée de manière à être installée par voie chirurgicale au moyen de broches dans les canaux médullaires du fémur et du tibia.

Un problème posé par ce type de prothèse réside dans le fait que les charges de compression tendent à user rapidement les constituants de l'articulation ou à produire des particules et de la poussière métalliques (à partir d'un ou de plusieurs éléments métalliques de l'articulation) qui deviennent la cause de complications et d'infections.

En effet, lorsque l'axe d'articulation de la prothèse est immobilisé en rotation et en translation par rapport à la chape, les mouvements relatifs entre les éléments de la prothèse sont constitués par des glissements en rotation entre les surfaces cylindriques ou planes en contact. Les forces de frottement ainsi mises en jeu, importantes et proportionnelles au poids du porteur de la prothèse, conduisent rapidement à une usure de l'articulation et à une perte de sa précision de fonctionnement. Un remède partiel à l'inconvénient précité réside dans l'emploi de paliers de frottement polymères car ils réduisent le risque de complications d'ordre infectieux. Cependant, leurs faibles résistances à l'usure et au fluage conduisent rapidement à une perte de la précision d'ajustage et de fonctionnement de la prothèse.

L'invention a pour objet une prothèse d'articulation qui fait application d'un pivot de flexion à organes roulants dont les composants présentent une résistance élastique indispensable au bon fonctionnement de la prothèse, dans ses zones en contact mutuel soumises à des pressions de Hertz.

Un autre objet de l'invention est un pivot d'articulation préassemblé susceptible d'être incorporé rapidement dans une prothèse existante, dans le but d'éviter la répétition de la totalité du processus chirurgical de mise en place de la prothèse ou des lésions des ligaments nécessaires à l'amortissement des efforts et offrant un guidage supplémentaire à l'articulation.

Un autre but de l'invention est de créer une prothèse d'articulation du genou simple à fabriquer et à monter qui permet avec une précision satisfaisante les divers mouvements fonctionnels de l'articulation.

Le problème est résolu par les caractéristiques de la seconde partie de la revendication 1.

Un mode de réalisation préféré de l'invention est plus complètement décrit ci-après en regard des dessins schématiques annexés, dans lesquels :

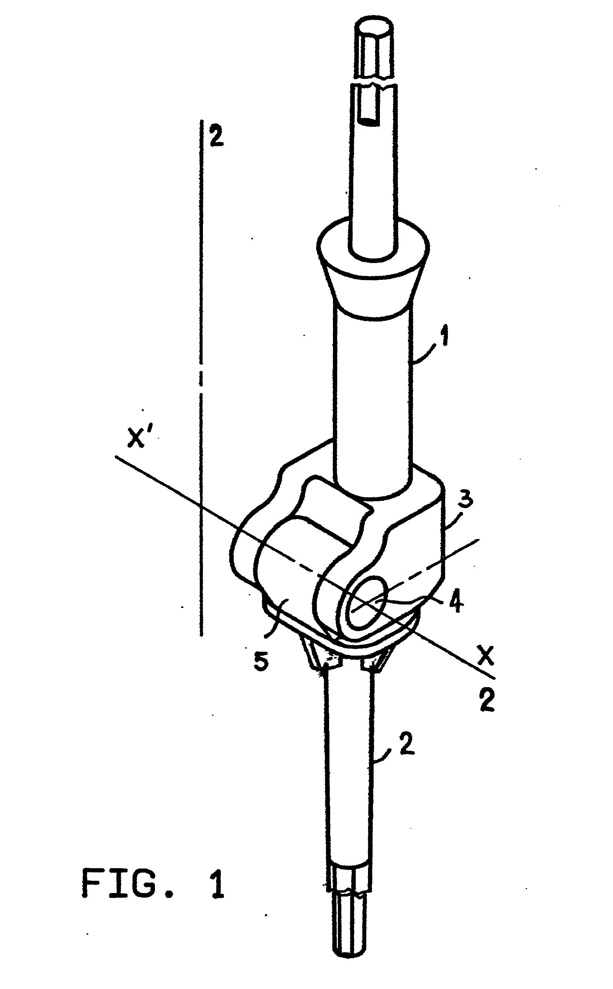

– la figure 1 est une vue en perspective de la prothèse d'articulation du genou en position allongée,
– la figure 2 est une vue en coupe partielle de l'articulation suivant la ligne 2–2 de la figure 1,
– la figure 3 est une vue en coupe du pivot de flexion suivant la ligne 3–3 de la figure 2.

La prothèse d'articulation du genou implantable décrite ci-après se compose d'un élément 1 en forme de broche destinée à être fixée dans le canal médullaire du tibia et d'un élément 2 en forme de broche destinée à être fixée dans le canal médullaire du fémur.

L'élément 1 porte une chape 3 traversée par l'axe XX' d'un pivot de flexion 4.

L'élément 2 porte un œil 5 introduit entre les branches de la chape 3 et est traversé par le même axe XX' du pivot 4.

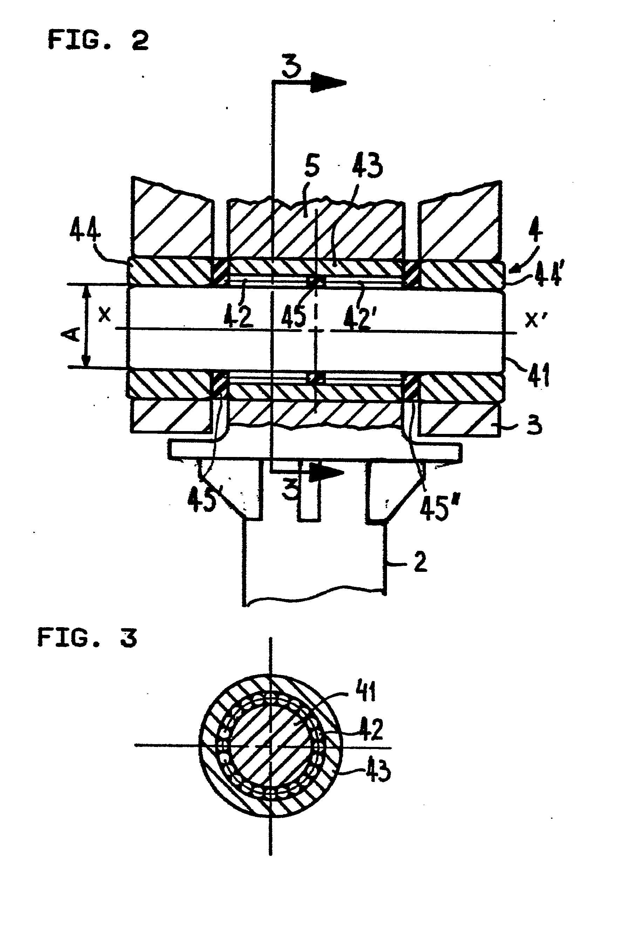

Le pivot 4 est constitué ainsi que cela est montré à la figure 2 par un axe solide 41 de diamètre A sur lequel roulent sans glisser deux ensembles de corps roulants 42, 42' constitués à titre d'exemple par des aiguilles cylindriques séparées par une rondelle en polyéthylène 45 entourées par une même bague 43 ajustée dans l'œil 5 de l'articulation.

Il y a lieu de noter que la géométrie des corps roulants n'est pas limitée au seul exemple décrit et pourrait tout aussi bien concerner des billes, rouleaux cylindriques ou coniques ou des combinaisons de tels corps.

La bague 43 est disposée entre deux butées latérales 44, 44' de même diamètre que celui de la bague 43, fixées solidairement aux extrémités de l'axe 41 et ajustées dans la chape 3 dont la fonction est d'assurer le guidage et l'immobilisation axiale de la bague par rapport à l'axe 41.

Deux autres rondelles de séparation en polyéthylène 45', 45" montées avec jeu sur l'axe 41 mais possédant de préférence un diamètre légèrement inférieur au diamètre de la bague 43, sont disposées entre les faces latérales respectives de la bague 43 et de chacune des butées 44, 44' pour favoriser un frottement doux lors des déplacements angulaires entre la bague 43 et les butées 44, 44'.

Dans le mode de réalisation représenté, les butées 44, 44' sont rigidement montées sur l'axe 41 par contact mécanique entre l'alésage des butées et la surface de l'axe 41 mais tout autre moyen de fixation peut être envisagé. Ces butées protègent le roulement des contraintes appliquées au cours du processus chirurgical de montage de la prothèse.

Les matériaux utilisés pour la fabrication des constituants principaux du pivot tels que l'axe 41, les corps roulants 42, 42', la bague 43, les butées latérales 44, 44' sont des alliages résistant à la corrosion du milieu biologique humain sans réactions avec les autres matériaux constitutifs de la prothèse. On utilisera de préférence un alliage de cobalt-chrome-molybdène-nickel. Par ailleurs, l'axe 41, les corps roulants 42, 42', la bague 43 sont traités spécialement dans le but d'obtenir une résistance mécanique suffisante aux opérations de montage et d'assemblage et aux pressions de contact dus aux efforts appliqués sur la prothèse au cours de son fonctionnement.

A titre indicatif, la dureté H $_{RC}$ de l'alliage déterminée par essai Rockwell utilisant un pénétrateur conique en diamant sera supérieure au nombre 50 et sa limite élastique sera supérieure à 1800 MPa.

A titre d'exemples, on pourra employer indifféremment l'un ou l'autre des alliages suivant A ou B définis ci-après :

## ALLIAGE A

| | |
|---|---|
| Cobalt | 29,5 à 39 % |
| Nickel | 33 à 37 % |
| Chrome | 19 à 21 % |
| Molybdène | 9 à 10,5 % |
| Fer | max 1 % |
| Titane | max 1 % |
| Carbone | max 0,025 % |
| Manganèse | max 0,15 % |
| Silicium | max 0,15 % |
| Phosphore | max 0,015 % |
| Soufre | max 0,010 % |

## ALLIAGE B

| | |
|---|---|
| Cobalt | 31 à 50 % |
| Nickel | 15 à 18 % |
| Chrome | 18,5 à 21,5 % |
| Molybdène | 6,5 à 7,5 % |
| Fer | 9 à 20 % |
| Beryllium | max 0,001 % |
| Carbone | max 0,15 % |
| Manganèse | 1 à 2 % |

Ces alliages sont écrouis à froid puis traités thermiquement pour leur conférer la résistance élastique souhaitée.

Le pivot précité est immobilisé axialement dans la chape 3 ou dans l'œil 5 par exemple au moyen d'une vis portée au choix par la chape 3 et immobilisant l'une quelconque des butées 44, 44' ou par l'œil 5 et immobilisant la bague 43. On notera néanmoins que d'autres procédés d'immobilisation peuvent être envisagés tels que le collage ou le sertissage sans sortir pour autant du cadre de l'invention.

## Revendications

1. Prothèse d'articulation du genou du type comportant un premier et un second éléments (1, 2) capables d'être assujettis rigidement aux os reliés par ladite articulation, le premier élément (1) portant une chape (3) traversée par un pivot (4) de flexion, le second élément (2) portant un oeil (5) introduit entre les branches de ladite chape (3), et dans laquelle le pivot (4) est constitué par un axe (41) portant extérieurement une bague (43) ajustée dans l'oeil (5) de l'articulation et des butées (44, 44') latérales d'un diamètre correspondant à celui de la bague (43) ajustées dans la chape (3), caractérisée par le fait que la bague (43) est montée sur des corps roulants (42, 42') disposés entre des rondelles de polyéthylène (45', 45") de séparation latérales placées au moins entre les faces latérales respectives de la bague (43) et de chacune des butées (44, 44').

2. Prothèse d'articulation selon la revendication 1, caractérisée par le fait que les constituants principaux (41, 42, 42', 43, 44, 44') du pivot (4) ont une dureté Rockwell H $_{RC}$ supérieure à 50 et une limite élastique supérieure à 1800 MPa.

3. Prothèse d'articulation selon la revendication 2, caractérisée par le fait que la matière des constituants principaux du pivot est un alliage métallique dont la composition est :
Cobalt 29,5 à 39
Nickel 33 à 37%
Chrome 19 à 21%
Molybdène 9 à 10,5%
Fer max 1%
Titane max 1%
Carbone max 0,025%
Manganèse max 0,15%
Silicium max 0,15%

EP 0 265 325 B1

Phosphore max 0,015%
Soufre max 0,010%

4. Prothèse d'articulation selon la revendication 2, caractérisée par le fait que la matière des constituants principaux du pivot est un alliage métallique dont la composition est :

Cobalt 31 à 50%
Nickel 15 à 18%
Chrome 18,5 à 21,5%
Molybdène 6,5 à 7,5%
Fer 9 à 20%
Beryllium max 0,001%
Carbone max 0,15%
Manganèse 1 à 2%

**Patentansprüche**

1. Kniegelenkprothese, mit einem ersten und einem zweiten Teil (1, 2), die in steifer Weise in den durch das Gelenk miteinander zu verbindenden Knochen zu befestigen sind, wobei das erste Teil einen Bügel (3) trägt, der von einem Drehzapfen (4) durchsetzt ist, das zweite Teil eine Öse (5) aufweist, die zwischen die Seitenwände des Bügels (3) eingesetzt ist und wobei der darin eingesetzte Zapfen (4) aus einer Welle (41) besteht, die einen Außenring (43) trägt, der in die Öse (5) des Gelenkes eingesetzt ist, sowie seitliche Anschläge (44, 44') trägt, deren Durchmesser demjenigen des Ringes (43) entsprechen und die in den Bügel (3) eingesetzt sind, dadurch gekennzeichnet, daß der Ring (43) auf Rollkörpern (42, 42') angeordnet ist, die zwischen Unterlegscheiben (45', 45'') aus Polyethylen zur seitlichen Abstandswahrung angeordnet sind und die wenigstens zwischen den entsprechenden Seitenflächen des Ringes (43) und jeder der Anschläge (44, 44') vorgesehen sind.

2. Gelenkprothese nach Anspruch 1, dadurch gekennzeichnet, daß die Hauptbestandteile (41, 42, 42', 43, 44, 44') des Zapfens (4) eine Rockwell Härte $H_{RC}$ von mehr als 50 aufweisen und eine obere elastische Grenze von 1800 MPa.

3. Gelenkprothese nach Anspruch 2, dadurch gekennzeichnet, daß der Stoff der Hauptbestandteile des Zapfens eine metallische Legierung mit der folgenden Zusammensetzung ist:

Kobalt 29,5 bis 39%
Nickel 33 bis 37%
Chrom 19 bis 21%
Molybdän 9 bis 10,5%
Eisen max 1%
Titan max 1%
Kohlenstoff max 0,025%
Mangan max 0,15%
Silizium max 0,15%
Phosphor max 0,015%
Schwefel max 0,010%.

4. Gelenkprothese nach Anspruch 2, dadurch gekennzeichnet, daß der Stoff der Hauptbestandteile des Zapfens eine metallische Legierung mit der folgenden Zusammensetzung ist:

Kobalt 31 bis 50%
Nickel 15 bis 18%
Chrom 18,5 bis 21,5%
Molybdän 6,5 bis 7,5%
Eisen 9 bis 20%
Beryllium max 0,001%
Kohlenstoff max 0,15%
Mangan 1 bis 2%.

**Claims**

1. A knee joint prosthesis of the type comprising first and second elements (1, 2) which are capable of being rigidly secured to the bones connected by said joint, the first element (1) bearing a yoke (3) through which passes a flexural pivot (4), the second element (2) bearing an eye (5) which is introduced between the limbs of said yoke (3), and in which the pivot (4) is formed by a shaft (41) which externally bears a sleeve (43) which is fitted into the eye (5) of the joint and lateral stops (44, 44') of a diameter corresponding to that of the sleeve (43) and fitted into the yoke (3) characterised in that the sleeve (43) is mounted on rolling bodies (42, 42') disposed between lateral polyethylene separating washers (45', 45'') which are disposed at least between the respective side faces of the sleeve (43) and each of the stops (44, 44').

2. A joint prosthesis according to claim 1 characterised in that the main components (41, 42, 42', 43,

4

44, 44′) of the pivot (4) are of a Rockwell hardness $H_{RC}$ of higher than 50 and have an elastic limit which is higher than 1800 MPa.

3. A joint prosthesis according to claim 2 characterised in that the material of the main components of the pivot is a metal alloy of the following composition:

Cobalt 29.5 to 39%
Nickel 33 to 37%
Chromium 19 to 21%
Molybdenum 9 to 10.5%
Iron max 1%
Titanium max 1%
Carbon max 0.025%
Manganese max 0.15%
Silicon max 0.15%
Phosphorus max 0.015%
Sulphur max 0.010%.

4. A joint prosthesis according to claim 2 characterised in that the material of the main components of the pivot is a metal alloy of the following composition:

Cobalt 31 to 50%
Nickel 15 to 18%
Chromium 18.5 to 21.5%
Molybdenum 6.5 to 7.5%
Iron 9 to 20%
Beryllium max 0.001%
Carbon max 0.15%
Manganese 1 to 2%.

FIG. 1

FIG. 2

FIG. 3